# EUROPÄISCHE PATENTSCHRIFT

(11) **EP 2 655 219 B1**
(45) Veröffentlichungstag und Bekanntmachung des Hinweises auf die Patenterteilung: **15.10.2014**
(21) Anmeldenummer: 11804496.5
(22) Anmeldetag: 16.12.2011
(51) Int. Cl.: B65D 90/02, C12M 1/00, C12M 3/00, B65D 90/06, B65D 90/08, C12M 1/107

(54) **SYSTEM MIT EINER BEHÄLTERWAND FÜR EINEN VON EINER FOLIE ABGEDECKTEN BEHÄLTER**
SYSTEM WITH A CONTAINER WALL FOR A CONTAINER COVERED BY A FILM
SYSTEME AVEC UNE PAROI DE CONTENANT POUR UN CONTENANT RECOUVERT PAR UN FILM

(30) Priorität: 22.12.2010 DE 102010055527
(43) Veröffentlichungstag der Anmeldung: 30.10.2013
(73) Patentinhaber: MT-Energie GmbH, 27404 Zeven (DE)
(72) Erfinder: BEHRENS, Jan, C., F., 27404 Seedorf (DE)
(74) Vertreter: Hauck Patent- und Rechtsanwälte
(86) Internationale Anmeldenummer: PCT/EP2011/006375
(87) Internationale Veröffentlichungsnummer: WO 2012/084161

(56) Entgegenhaltungen:
- EP-A1- 1 882 734
- DE-A1- 2 249 057
- DE-A1- 2 711 146
- DE-B- 1 064 869
- DE-U1-202008 016 776

## Beschreibung

Die Erfindung bezieht sich auf ein System nach Anspruch 1.

Behälter zum Lagern und Gären von Gärstoffen, beispielsweise in sogenannten Biogasanlagen, haben erhebliche Abmessungen. Sie sind zumeist siloartig ausgebildet und haben dementsprechend zumeist eine kreisrunde Grundfläche. Die Wand ist z.B. aus Beton gegossen, und die Abdichtung nach oben erfolgt oftmals mit Hilfe einer dachförmigen Folie, die durch Gasentwicklung oder durch Einfuhr von Druckluft in einem gespannten und angehobenen Zustand gehalten wird. Um die Folie am Rand wirksam an der Behälterwandung festzulegen, ist auch bekannt, im oberen Außenbereich der Wand ein Klemmprofil anzubringen, in welches der Folienrand hineingelegt und mit Hilfe eines Klemmschlauchs festgelegt wird.

Aus DE 10 64 869 A ist bekannt geworden, den Randbereich einer einen Behälter nach oben abdichtenden Folie in einen umlaufenden im Querschnitt U-förmigen nach oben offenen Schlitz einzulegen und anschließend einen aufblasbaren Schlauch einzulegen, um den Folienrand festzulegen. Aus DE 10 2006 035 227 B3 ist bekannt geworden, sowohl eine Gasspeicher- als auch Abdeckhaube aus Folienmaterial in einer C-förmigen außen am Behälter angebrachten Schiene festzulegen, wobei ein Klemmschlauch in die Schiene eingelegt wird zur Fixierung der Folien innerhalb der Schiene.

Je nach dem im Behälter herrschenden Gasdruck, Stützluftdruck oder den äußeren Einflüssen werden über die Folien dementsprechende Zugkräfte in die Schiene eingebracht. Es besteht daher die Gefahr, dass durch Verformung des Klemmprofils oder mangelnde Klemmkraft des Klemmschlauchs die Folie aus der Schiene herausrutscht.

Diesem kann in der Praxis durch festes Verflanschen der Folie(n) mit dem Behälter auch entgegengewirkt werden. Die Folie kann bis zum Zerreißen belastet werden. Nachteilig ist der erhöhte Aufwand beim Öffnen der Abdeckung, z.B. zu Wartungszwecken. Außerdem kann die Abdeckung beim Reißen der Folie ihre Funktion verlieren, was vermieden werden muss.

Aus WO 2010/075981 A2 ist eine Behälterwand für einen von einer Folie abgedeckten Behälter bekannt geworden, bei der ein Klemmkanal für eine Kunststofffolie, welche den Behälter an der Oberseite abdeckt, mit einem durch einen oberen und einen unteren Vorsprung begrenzten verengten, nach außen offenen Eintrittsschlitz versehen ist. In den Eintrittsschlitz wird ein Einlageprofil eingesetzt, das in Schrägstellung in den Klemmkanal einführbar ist und in annähernd paralleler Lage zu den Vorsprüngen gegen ein Herausbewegen aus dem Klemmkanal durch die Vorsprünge gehindert ist. Der Klemmkanal kann in das Material der Behälterwand eingeformt oder eine separate Profilschiene sein, die entweder auf die Behälterwand aufgesetzt oder in diese eingeformt wird. Durch die Vorsprünge sind zwei Hinterschnitte gebildet, die unabhängig davon, ob ein separates Klemmprofil verwendet wird oder der Klemmkanal in das Material der Behälterwand geformt wird, relativ aufwendig zu fertigen sind. Zudem wird durch die zwei Vorsprünge das Einführen des Einlageprofils in den Klemmkanal erschwert bzw. nur durch eine besondere Formgebung des Klemmkanals ermöglicht.

Die DE202008016776 U1 offenbart eine Vorrichtung zur Festlegungs einer Folie an einem Behälter.

Es ist auch bekannt, den unteren Schenkel eines Klemmprofils separat zu formen und ihn schwenkbar oder verschiebbar am übrigen Profil anzubringen. Damit ist die Möglichkeit geschaffen, den Eintrittsschlitz für den Klemmkanal zur Montageerleichterung zu vergrößern.

Der Erfindung liegt die Aufgabe zugrunde, ein System mit einer Behälterwand für einen Behälter mit einer Vorrichtung zur Festlegung einer den Behälter an der Oberseite dachförmig abschließenden Folie aus Kunststoff zu schaffen, das noch einfacher herstellbar und montierbar ist.

Diese Aufgabe wird durch die Merkmale des Patentanspruchs 1 gelöst.

Bei dem System mit der erfindungsgemäßen Behälterwand ist der verengte Eintrittsschlitz nur durch einen oberen Vorsprung begrenzt, während die annähernd ebene untere Wand des Klemmkanals vorsprunglos in den Eintrittsschitz übergeht.

Bei der Erfindung wurde erkannt, dass es zur Festlegung des Einlegeprofils im Klemmkanal ausreicht, wenn das Einlageprofil bei Zugspannung an der Folie gegen den oberen Vorsprung von innen anliegt und das Einlageprofil sich auf der unteren Wand des Klemmkanals abstützt. Ein Herausrutschen des Einlageprofils mit der unteren Kante ist nicht zu befürchten. Lediglich bei der Montage kann es erforderlich sein, nach dem Einsetzen des Einlageprofils mit herumgelegter Folie dafür zu sorgen, dass die untere Kante des Einlageprofils nicht über den Eintrittsschlitz nach außen rutscht, solange noch keine Spannung auf die Folie aufgebracht worden ist. Hierfür stehen jedoch einfache Mittel zur Verfügung.

Die Herstellung eines Klemmkanals mit nur einem oberen Vorsprung ist fertigungstechnisch signifikant einfacher als die eines Klemmkanals, bei dem der Eintrittsschlitz von einem oberen und einem unteren Vorsprung gebildet ist. Außerdem ist das Einsetzen des Einlageprofils mit herumgelegter Folie in den erfindungsgemäßen Klemmkanal entschieden einfacher zu bewerkstelligen als bei dem bekannten Klemmprofil. Ferner kann die Formgebung des Klemmkanals stark vereinfacht werden. Die untere Wand des Klemmkanals braucht nicht durchgehend zu sein, sondern kann aus in Längsrichtung des Klemmkanals beabstandeten Wandabschnitten bestehen.

Das erfindungsgemäße System mit der Behälterwand ist vorzugsweise aus Beton hergestellt und im oberen Bereich ist der nach außen offene Klemmkanal vorgesehen mit dem vorzugsweise seitlichen Eintrittsschlitz, dessen Höhe kleiner ist als die Höhe eines in den Klemmkanal einsetzbaren länglichen Einlageprofils, um welche die Folie herumlegbar ist. Das Einlageprofil wird mit herumgeschlungener Folie zum Eintrittsschlitz gekippt über diesen in dem Klemmkanal eingeführt und anschließend z. B. in eine vertikale Lage gebracht, so dass es bei einer Zugkraft an der Folie von innen gegen den oberen Vorsprung, welcher den Eintrittsschlitz begrenzt, angedrückt wird. Die untere Kante des Einlageprofils steht auf der annähernd ebenen unteren Wand des Klemmkanals auf. Mit Hilfe einer derartigen Konstruktion lassen sich hohe Zugkräfte auffangen, ohne dass Gefahr besteht, dass die Folie aus dem Klemmkanal herausgleitet.

Um die Folie bei zu hohen Kräften vor dem Zerreißen zu schützen, kann nach einer Ausgestaltung der Erfindung das Einlageprofil eine Sollbruchstelle aufweisen, wobei es im Wesentlichen um eine horizontale Achse knickt, wenn auf die Folie eine vorgegebene maximale Kraft wirkt. Nach einer weiteren Ausgestaltung der Erfindung kann das Einlageprofil aus einem Material hergestellt sein, das bei Erreichen einer maximal zulässigen Kraft auf die Folie durch Eigenverformung die kraftschlüssige Verbindung der Folie lockert oder löst. Das Einlageprofil wird vorzugsweise aus Strangguss oder Flachmaterial, welches beispielsweise aus Metall, Gummi oder Kunststoff besteht, hergestellt. Es kann als flacher Streifen oder gebogen oder geknickt geformt sein oder ein mehr oder weniger flacher Profilstreifen sein. Der flache Streifen kann einen rechteckigen, trapezförmigen oder winkligen Querschnitt haben.

Je nach den Druckverhältnissen im Behälter und der an der Dachfolie angreifenden Windkraft werden erhebliche Zugkräfte auf den Folienrand ausgeübt. Daher besteht die Gefahr, dass der Folienrand herausgezogen wird, wenn die Zugkräfte einen bestimmten Wert übersteigen. Diese Gefahr wird erhöht, wenn der Winkel des Folienrandes zum zugeordneten Schenkel eines schienenförmigen Klemmprofils einen größeren Wert annimmt, so dass der Schenkel eine Verformung erleidet und nach oben gebogen und der Kraftschluss zwischen Folienrand und Klemmprofil reduziert wird.

Bei einer Ausgestaltung der Erfindung ist dafür gesorgt, dass insbesondere bei höherer Zugbelastung der Folie die auf den zugewandten oberen Schenkel einwirkende Zugkraft im Wesentlichen als Druckkraft in den Schenkel eingeleitet wird und nur als kleineres Biegemoment auf den Schenkel wirkt. Es kommt daher nicht zu einer nachteiligen Verformung des Klemmprofils mit der Folge, dass der Folienrand herausgezogen wird.

Der Folienrand erstreckt sich z.B. in einem Winkel von 30° bis 45° zur Horizontalen. Der dem Folienrand zugekehrte Schenkel des Klemmprofils erstreckt sich vorzugsweise im gleichen Winkel. Hierdurch kann im Vergleich zu einem horizontalen Schenkel mehr Kraft von der Folie in die Schiene eingeleitet werden, bevor die Schiene die kraftschlüssige Verbindung durch Verformung lockert. Somit ist bei der Erfindung auch bei höheren Auszugskraftwerten eine sichere Verankerung des Folienrands in dem Klemmprofil gewährleistet.

Eine derartige Profilschiene kann außen an der Behälterwand befestigbar sein. Hierfür gibt es verschiedene Konstruktionsmöglichkeiten. Eine besteht nach einer Ausgestaltung der Erfindung darin, dass außen an die Behälterwand in Abständen vertikale Befestigungsprofile anbringbar sind, an welchen die Profilschiene vorzugsweise durch Verschweißung befestigt ist. Die Schiene, die vorzugsweise aus Längsabschnitten besteht, die anschließend miteinander verbunden werden, wird zunächst gebogen und anschließend mit den Befestigungsprofilen kraftschlüssig verbunden. Diese werden danach mit der Behälterwand verbunden, beispielsweise durch geeignete Anker. Die Behälterwand kann hierbei aus verschiedensten Materialien bestehen, so z.B. Beton, Stahl, Kunststoff oder Holz bzw. aus einer Kombination der verschiedenen Materialien bestehen.

In einer weiteren Ausgestaltung der Erfindung ist in diesem Zusammenhang vorgesehen, dass das Befestigungsprofil im Querschnitt U-förmig ist, wobei der Steg des Befestigungsprofils mit der Behälterwand verbindbar ist.

Ein Klemmprofil in Form einer Profilschiene kann in die Behälterwand eingegossen sein. Auch bei dieser Lösung kann eine hohe Auszugskraft erzielt werden, wenn das Klemmprofil in einem bestimmten Abstand zur oberen Kante der Behälterwand angeordnet ist. Eine Verformung des dem Folienrand zugekehrten Schenkels der Schiene ist nicht zu befürchten.

In einer weiteren Ausgestaltung der Erfindung ist die obere Kante oder der obere Schenkel der Profilschiene mit der Oberseite der Behälterwand bündig. Die Oberseite der Behälterwand kann nach einer weiteren Ausgestaltung der Erfindung zur Außenseite hin ansteigen.

Ist das Klemmprofil bzw. die Profilschiene mit Abstand zur Oberseite der Behälterwand in diese eingegossen ist nach einer weiteren Ausgestaltung der Erfindung die äußere obere Kante der Wand gerundet. Auf diese Weise wird die Folie nicht so stark beansprucht.

Bei einer Einformung einer Profilschiene in das Material der Behälterwand braucht die Dicke des Materials der Profilschiene, die vorzugsweise aus Flachmaterial oder Strangguss geformt ist, nur sehr gering sein, da die Kräfte weitgehend von dem umgebenden Material der Behälterwand aufgefangen werden.

Die erfindungsgemäße Maßnahme bewirkt, dass nach dem Spannen der Folie das Einlageprofil sich fest gegen die untere Wand abstützt. Die Gefahr, dass das Einlageprofil über den Eintrittsschlitz nach außen bewegt wird, besteht nicht. Diese Gefahr ist möglicherweise aber gegeben bei der Montage kurz nach dem Einsetzen des Einlageprofils und vor dem Spannen der Dachfolie. Die Gefahr besteht außerdem, wenn aus anderen Gründen die Spannung an der Dachfolie nachlässt oder aufgegeben ist. Daher sieht eine Ausgestaltung der Erfindung vor, dass die untere Wand nahe dem Eintrittsschlitz des Klemmkanals in Längsabschnitten Erhebungen aufweist, die entfernbar oder beweglich so angeordnet sind, dass sie in einer ersten Position ein Widerlager für das im Klemmkanal befindliche Einlageprofil bilden und in einer zweiten Position die dem Klemmkanal zugekehrte Fläche der unteren Wand freigeben. Vor der Montage des Einlageprofils mit der darum gelegten Folie ist die dem Klemmkanal zugekehrte Fläche der unteren Wand völlig frei, sodass das Einsetzen des Einlageprofils mit der Folie problemlos bewerkstelligt werden kann. Anschließend wird nach der beschriebenen Ausführungsform der Erfindung eine Reihe von Widerlageelementen bzw. Erhebungen montiert, die verhindern, dass die untere Kante des Anlageprofils aus dem Eintrittsschlitz herausrutscht.

Es sind verschiedene konstruktive Möglichkeiten denkbar, die beschriebene Ausführungsform zu realisieren. Nach einer Ausgestaltung der Erfindung sind in Öffnungen der unteren Wand einsteckbare oder einschraubbare Widerlagerelemente vorgesehen. Diese können z.B. aus Kunststoff bestehen.

Nach einer weiteren Ausgestaltung der Erfindung sind an der unteren Wand gelagerte Klappmechanismen vorgesehen. Die Klappmechanismen sind so ausgebildet, dass sie in einer abgeklappten Stellung ein freies Einführen des Einlageprofils erlauben und in der hochgeklappten Position ein Widerlager bzw. ein Hindernis für das Herausbewegen des Einlageprofils aus dem Eintrittsschlitz bilden. Schließlich können auch von vom auf die untere Wand aufklemmbare Klemmelemente vorgesehen werden, um ein Widerlager zu bilden. Es sei jedoch nochmals betont, dass die beschriebenen Widerlager und Erhebungen für die Funktion der Festlegung der Folie im Klemmprofil nicht notwendig sind. Sie sind lediglich Hilfsmittel für die Montage und für den Fall, dass ein Herausrutschen des Klemmprofils aufgrund mangelnder Spannung in der Folie befürchtet werden muss.

Ausführungsbeispiele der Erfindung werden nachfolgend anhand von Zeichnungen näher erläutert.
- Fig. 1a: zeigt schematisch die Draufsicht auf einen Behälter.
- Fig. 1b: zeigt einen Schnitt durch die Darstellung nach Fig. 1 entlang der Linie 2-2.
- Fig. 2: zeigt eine Einzelheit III nach Fig. 1b zur Festlegung einer Dachfolie.
- Fig. 3: zeigt eine weitere Ausführungsform der Festlegung einer Dachfolie.
- Fig. 4: zeigt eine weitere Ausführungsform zur Festlegung einer Dachfolie.
- Fig. 5: zeigt eine weitere Ausführungsform zur Festlegung einer Dachfolie.
- Fig. 6: zeigt eine weitere Ausführungsform zur Festlegung einer Dachfolie.
- Fig. 7: zeigt eine weitere Ausführungsform zur Festlegung einer Dachfolie.
- Fig. 8: zeigt eine weitere Ausführungsform der Erfindung zur Festlegung einer Dachfolie.
- Fig. 9a u. 9b: zeigen eine ähnliche Ausführungsform wie Fig. 8.
- Fig. 10: zeigt perspektivisch ein Klemmprofil mit verschiedenen Widerlagerelementen.
- Fig. 11: zeigt einen Schnitt durch ein Klemmprofil nach der Erfindung mit einem Klappmechanismus.
- Fig. 12: zeigt die Vorderansicht des Klemmprofils nach Figur 11.
- Fig. 13: zeigt eine weitere Ausführungsform eines Klemmprofils nach der Erfindung.

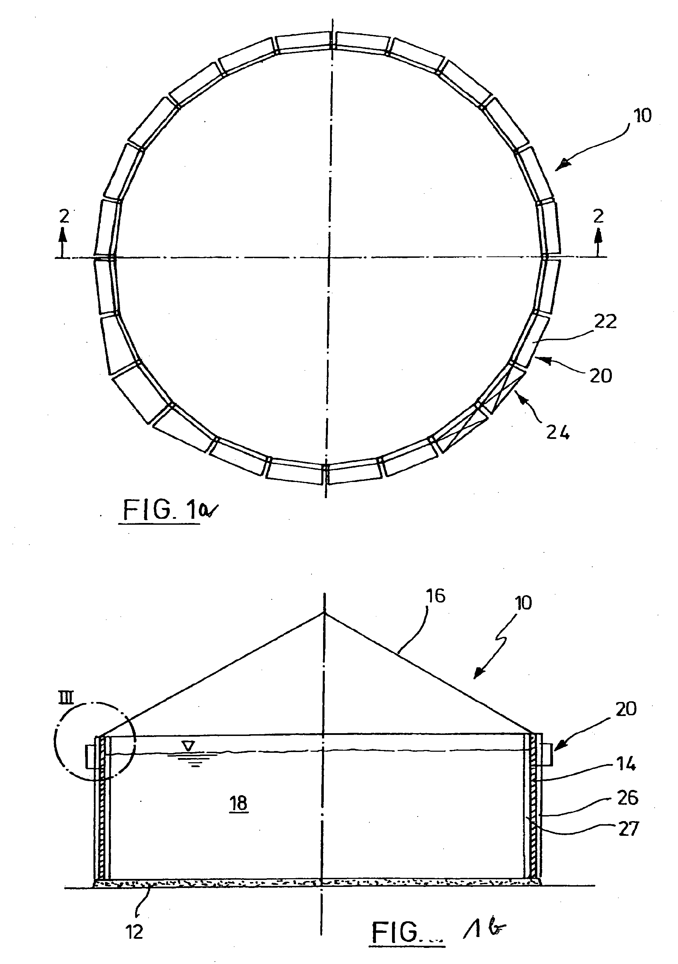

In den Fig. 1 a und b ist ein Behälter 10 angedeutet, der eine Grundplatte 12 aus Beton aufweist und eine zylindrische Wand 14, ebenfalls aus Beton. Die Betonwand wird z. B. mit Hilfe einer Vielzahl von außen und innen angeordneten Schalungselementen hergestellt. Nach oben spannt sich mindestens eine Kunststofffolie 16 über den Behälter 10, wobei ihr Rand im oberen Bereich an der Außenseite der Wand 14 festgelegt wird, wie weiter unten noch zu beschreiben sein wird. Im Behälter befindet sich z. B. ein Gärstoff 18. An der Außenseite der Wand 14 ist ein umlaufender Laufsteg 20 mit Geländer gezeigt. Der Laufsteg besteht aus einzelnen Laufstegelementen, die auch miteinander verbunden sein können. Der Laufsteg ist für die Erfindung nicht von Bedeutung. In Fig. 1b ist außerdem eine Außenschalung 26 und eine Innenschalung 27 angedeutet. Sie dient, wie erwähnt, zur Herstellung der Wand, wobei die Schalung nach Errichtung der Betonwand 14 entfernt wird.

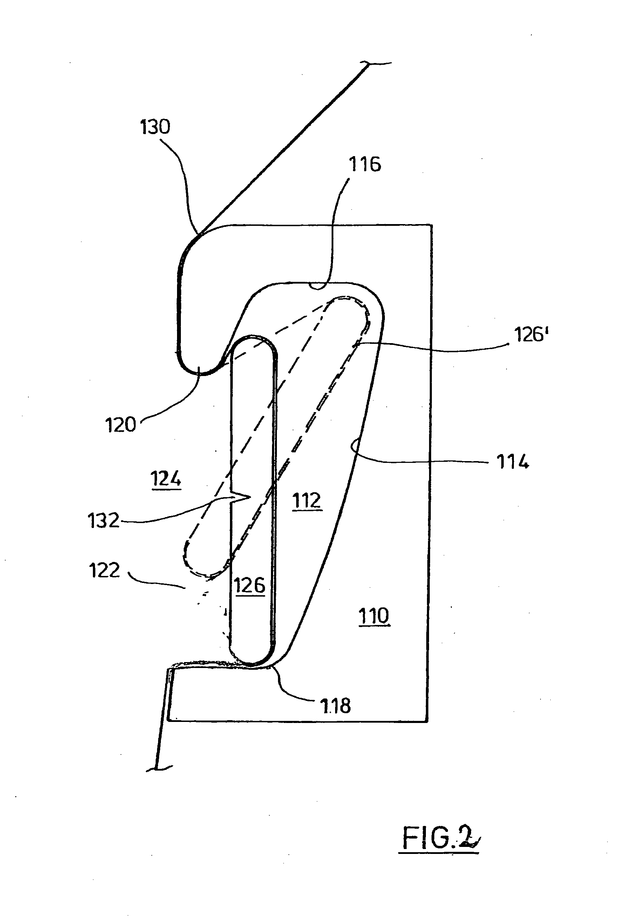

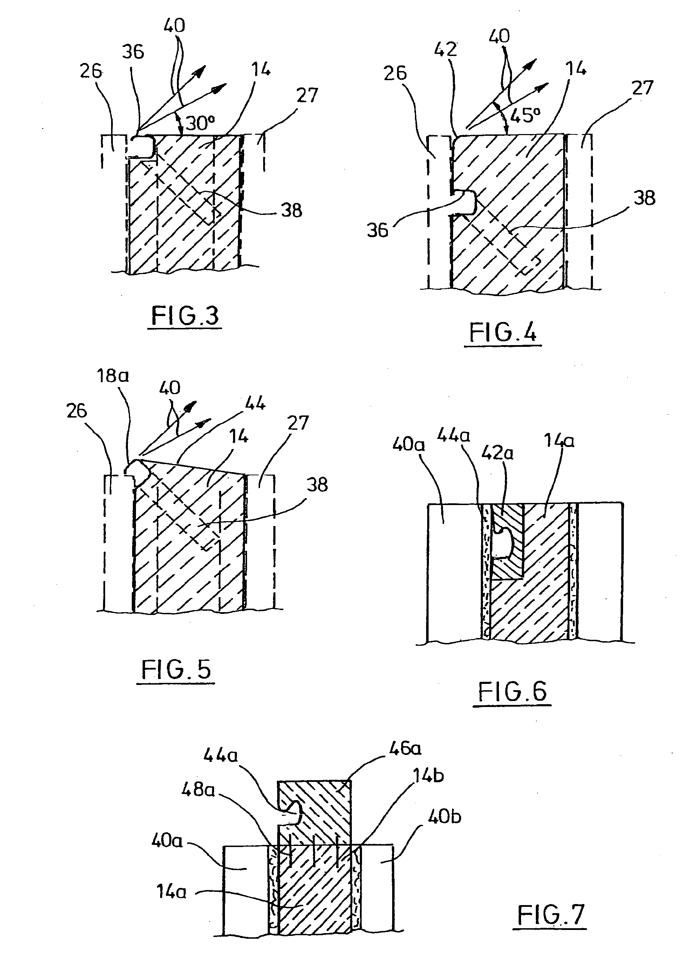

In Figur 2 ist bei 110 der obere Teil der Behälterwand dargestellt, wie sie etwa auch in Figur 3 zu erkennen ist. Sie enthält einen Klemmkanal 112, der einen Boden 114, eine obere Wand 116 und eine untere Wand 118 aufweist. An den Übergängen sind die Wände im Querschnitt gekrümmt. Durch nach unten weisenden Vorsprung 120 (im Querschnitt) ist ein verengter Eintrittsschlitz 124 zum Kanal 112 hin gebildet. Die untere Wand geht stetig in den Eintrittsschlitz 124 über. Ein Klemmelement in Form eines Flachprofils 126 hat über seine Höhe bzw. Dicke annähernd gleiche Abmessungen und ist an den Kanten gerundet. Die Breite des Flachprofils 126 ist größer als die Höhe des Eintrittsschlitzes 124 und ist so bemessen, dass er durch die Innenseite des Vorsprungs 120 und die Wand 118 gehalten wird, wenn das Flachprofil 126 an der unteren Wand 118 aufsetzt. In der gestrichelt gezeigten, gekippten Lage des Flachprofils 126, die bei 126' gezeichnet ist, kann diese über den Eintrittsschlitz 124 in die Nut 112 eingeführt werden. Zudem wird um das Flachprofil 126 eine Folie 130 geschlungen. Nach dem Einsetzen des Flachprofils 126 und dem Aufsetzen des Flachprofils 126 auf der Wandung 118 erfolgt durch einen Zug an der Folie 130 ein Zurückkippen des Flachprofils 126 in eine annähernd vertikale Position parallel zum Eintrittsschlitz. Dadurch ist die Folie 130 in dem Kanal 112 geklemmt und kann nicht herausgezogen werden.

Wie ferner zu erkennen, weist das Flachprofil 116 eine Einkerbung 132 auf, welche eine Sollbruchstelle bildet. Die Sollbruchstelle verhindert im Fall einer Havarie in letzter Instanz ein Reißen der Folie und kann für eine Notentlastung sorgen

Es versteht sich, dass der Klemmkanal 112 auch von z.B. einer separaten Profilschiene herstellbar ist, was weiter unten noch beschrieben wird. Für die Festlegung der Folie ist hierbei das Profil des Klemmkanals und das Klemmelement in dem Klemmkanal wesentlich.

In den Fig. 3 - 5 ist ein Schnitt durch den oberen Bereich der Behälterwandung 14 gezeigt, die jedoch im oberen Bereich unterschiedlich ausgestaltet ist, wie nachfolgend beschrieben wird. Man erkennt außerdem wiederum eine Außen- und Innenschalung 26, 27 nach Fig. 1b in Herstellung der Behälterwand aus Beton. Das Einlage- oder Flachprofil ist aus Darstellungsgründen fortgelassen.

In Fig. 3 ist eine kastenförmige Profilschiene 36 dargestellt, die beispielsweise eine Form hat, wie sie in Fig. 2 gezeigt ist. Wie erkennbar, ist die Profilschiene 36 in die Wand 14 so eingebracht, dass die obere Seite bzw. der obere Schenkel bündig mit der horizontalen Oberseite der Wand 14 ist. Bei der Herstellung wird die Profilschiene 36 mit der Außenschalung 26 lösbar verbunden (nicht gezeigt). Nach dem Formen der Wand 14 wird naturgemäß die Schalung 26, 27 entfernt, wobei zuvor die lösbare Verbindung zur Außenschale 26 getrennt wird. Die Profilschiene 36 ist dann mit der Wand 14 verbunden.

Die Profilschiene besteht aus einzelnen Abschnitten bestimmter Länge, die vorzugsweise deutlich kleiner ist als die Umfangslänge des Behälters 10. Dabei kann ein Schienenabschnitt mit einem oder mehreren benachbarten Schalungselementen verbunden sein. Die Profilschiene 36 bzw. ihre einzelnen Abschnitte sind mit Ankerabschnitten versehen, die, wie bei 38 gezeigt, schräg nach unten in die Wand 14 hineinragen. In Fig. 3 ist mit den Pfeilen 40 die Zugrichtung einer Folie angedeutet, die in Fig. 1b mit 16 bezeichnet ist. Sie ist in der Profilschiene 36 festgelegt. Dies wird weiter unten noch beschrieben. Die unterschiedlichen Winkel der Zugrichtung 40 ergeben sich aus der Art der Folie (Gasspeicherfolie, Traglufthaube) und konstruktiven Gegebenheiten. Je nach Winkelgröße verändern sich Druck und Biegekraft am oberen Schenkel der Profilschiene.

Die Ausführungsform nach Fig. 4 unterscheidet sich von der nach Fig. 3 dadurch, dass die Profilschiene 36 im Abstand zur Oberseite der Wand 14 eingebettet ist. Auch hier wird die Profilschiene 36 zunächst mit der Außenschalung 26 verbunden. Die Folie, die mit ihrem Rand in der Profilschiene 36 festgelegt wird, erstreckt sich zunächst vertikal an der Außenseite der Wand 14 und wird über eine gerundete Kante 42 der Wand 14 schräg nach oben geführt. Die Rundung 42 reduziert die Beanspruchung, die durch die Umlenkung der Folie stattfindet.

In Fig. 5 ist eine Profilschiene 18a ebenfalls so in die Wand 14 eingebettet, dass seine obere Kante mit der Oberseite der Wand 14 bündig ist. Diese Oberseite weist jedoch eine Steigung auf, wie bei 44 angedeutet. Die Steigung ist zur Außenseite hin gerichtet. Die Schiene 18a ist wiederum mit der Außenschalung 26 lösbar verbunden, wenn die Wand 14 hergestellt wird. Anschließend wird diese Verbindung gelöst, damit die Außenschalung 26 entfernt werden kann. Eine abgekippte Anbringung der Profilschiene hat den Vorteil, dass deutlich größere Auszugswiderstände für den Folienrand erzielt werden bzw. eine günstige Beanspruchung der Profilschiene. Der obere Schenkel wird im wesentlichen in seiner Erstreckungsrichtung beansprucht und weniger auf Biegung. Der Folienrand ist mithin besonders sicher in der Profilschiene 18a gehalten.

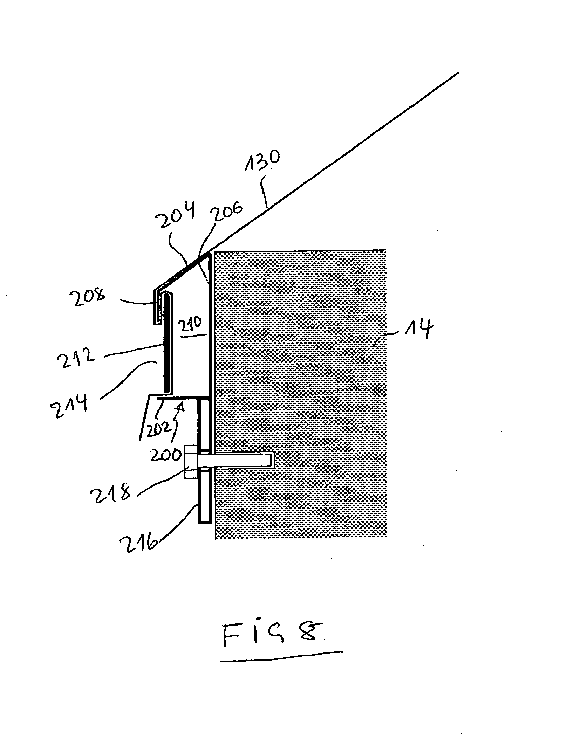

An die Behälterwand 14 in Fig. 8 ist eine Profilschiene 200 angebracht, die einen unteren horizontalen Wandabschnitt 202 aufweist, einen oberen dachförmigen Wandabschnitt 204 und einen vertikalen Stegabschnitt 206. Der schräge obere Wandabschnitt 204 geht in einen vertikalen nach unten ragenden Vorsprung 208 über. In den auf diese Weise gebildeten Klemmkanal 210 ist ein Flachprofil 212 eingesetzt, dessen Breite größer ist als die Höhe des Eintrittsschlitzes 214 in den Klemmkanal 210. Der Eintrittsschlitz wird gebildet durch die annähernd ebene untere Wand 202, die vorsprunglos in den Eintrittsschlitz 214 übergeht und den Vorsprung 208. Die Folie 130 (siehe auch Fig. 2) schlingt sich um den oberen Vorsprung 208 herum und um das Flachprofil 212, um im unteren Bereich des Eintrittsschlitzes 214 diesen nach unten zu verlassen. Bei einem Zug an der Folie 130 wird das Flachprofil 212 im oberen Bereich gegen den Vorsprung 208 angepresst und zugleich nach unten gegen die untere Wand 202 angedrückt, sodass auf diese Weise die Folie 130 wirksam in der Profilschiene 200 eingeklemmt ist. Die Schrägung der oberen Wand 214 entspricht dem Verlaufswinkel der Folie 130. Dies ist aber keine Voraussetzung. Im Prinzip könnte die obere Wand 204 auch horizontal verlaufen.

An der Unterseite der unteren Wand 202 sind in Abständen Halteabschnitte 216 aus Stahlblech oder dergleichen angebracht, die Löcher aufweisen, durch welche hindurch eine Ankerschraube 218 hindurchgeführt werden kann, um diese in der Betonwand 14 zu verschrauben. Die Profilschiene 200 bzw. Längsabschnitte von dieser, kann/können durch Kantung von Flachmaterial geformt werden. Es ist aber auch eine Herstellung im Strangpressverfahren denkbar.

Statt ein Klemmprofil in Form einer Profilschiene in die Wand einzubetten, kann ein Positiv-Profil aus Kunststoff mit der Schalung verbunden werden, das anschließend aus der Wand entfernt wird, z. B. durch Wärme oder ein Reagens. Danach verbleibt in der Wand ein Klemmprofil mit der gleichen Funktion wie der einen Klemmschiene.

In den Figuren 6 und 7 sind andere Möglichkeiten zur Bildung eines Klemmprofils angedeutet. In Fig. 6 ist ein Außchenschalungselement 40a lösbar mit einem Faserbetonteil 42a verbunden, wobei die lösbare Verbindung, die von außen zugänglich sein muss, nicht gezeigt ist. Das Faserbetonteil 42a weist einen vorgefertigten Kanal 44a auf und wird beim Gießen des Betons in diesen eingebettet (Wand 14a). Das Faserbetonteil 42a kann vorgefertigt sein. Es kann entfernt von der Baustelle mit dem Schalungselement 40a schon verbunden werden, bevor es zur Baustelle kommt oder an der Baustelle mit dem Schalungselement 40a verbunden werden.

Es sei nur der Vollständigkeit halber erwähnt, dass immer nur von einem Schalungselement bzw. einem Profilschalungsteil die Rede ist. Es versteht sich, dass in Umfangsrichtung gesehen eine Vielzahl von Außenschalungselementen 40a vorgesehen ist und auch eine Vielzahl von Schalungsprofilteilen 42a.

In Figur 7 ist eine andere Möglichkeit dargestellt. Hierbei ist auf die Oberseite der Betonwand 14b ein Betonelement 46a aufgesetzt, das mit einem Klemmkanal 44a versehen ist. Das Betonelement 46a ist vorgefertigt und weist eine nach unten und außen stehende Bewehrung 48a auf, die in den weichen Beton nach dem Gießen der Wand 14b eingesetzt werden kann, um das Betonelement 46a mit der Wand 14b zu verbinden. In diesem Fall sind die Schalungselemente 40a, 40b etwas niedriger als die Höhe der Behälterwand 14b, nämlich verringert um die Höhe des Fertigbetonelements 46a.

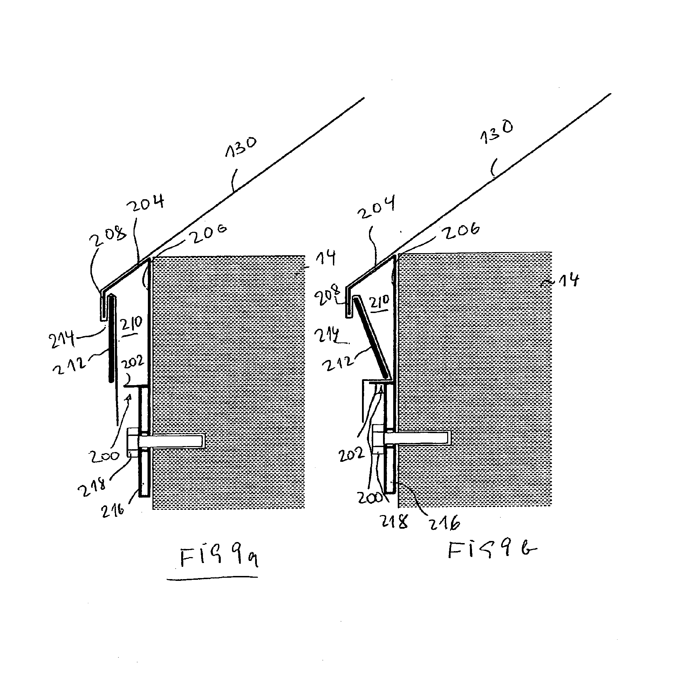

In Fig. 9a ist gezeigt, wie das Einlageprofil 212 in vertikaler Lage von unten in den Eintrittsschlitz 214 eingeführt wird. Im Klemmkanal wird das Einlageprofil 212 schräg gestellt mit seiner unteren Kante nahe der Wand 206 und mit der oberen Kante am Vorsprung 208 von innen anliegend. Ein Vorsprung bezüglich der Wand 202 ist nicht vorgesehen. Gleichwohl ist die herumgelegte Folie 130 ausreichend geklemmt und kann nicht herausrutschen.

Je nach Breite des Flachprofils 212 und der Breite der unteren Wand 202 muss es in Schrägstellung in den Schlitz 214 eingeführt werden, wie z.B. Fig. 8 erforderlich macht, vor allem wenn die untere Wand 202 bis in Höhe des Vorsprungs 208 erstreckt ist. In Fig. 9a und 9b ist die Wand 202 kürzer als der Abstand des Vorsprungs 208 von der Wand 14, was das vertikale Einführen des Einlageprofils 212 in den Schlitz 214 erlaubt. Anschließend wird es gemäß Fig. 9b schräg gestellt.

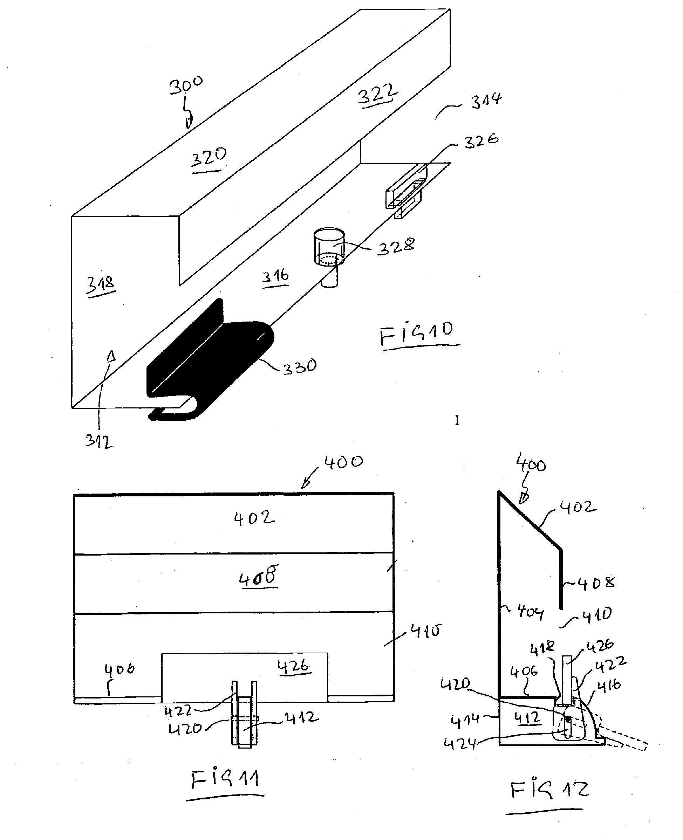

Figur 10 zeigt perspektivisch einen Teil eines Klemmprofils 300 mit einem Eintrittsschlitz 314. Der Klemmkanal 312 wird gebildet durch eine untere flache Wand 316, einer hinteren Wand 318, einer oberen Wand 320 und einem von der oberen Wand vom abgekanteten Abschnitt 322, der als Vorsprung den Eintrittsschlitz 314 von oben begrenzt.

In Öffnungen der unteren Wand 316 können Widerlagerelemente 326 bzw. 328 eingesteckt werden. Sie liegen nahe dem Eintrittsschlitz und können wahlweise entfernt werden. Das zylinderförmige Widerlagerelement 328 kann z.B. auch in ein Loch der unteren Wand 316 eingeschraubt werden. Das Widerlagerelement 326 ist flach und besitzt einen quaderförmigen Vorsprung, der in ein passendes Loch eingesteckt ist. Die Widerlagerelemente sind z.B. aus Kunststoff.

Ferner ist in Figur 10 ein Federklipp 330 gezeigt von U-förmigem Querschnitt, der von der vorderen Kante der unteren Wand 316 her auf diese aufgeklemmt werden kann. Der Klipp 330 weist am oberen Schenkel einen nach außen gekanteten Abschnitt 332 auf, der ebenfalls ein Widerlager für ein nicht gezeigtes Einlageprofil bildet, das über den Eintrittsschlitz 314 in den Klemmkanal 312 einsetzbar ist. Für dieses Einsetzen sind jedoch die gezeigten Widerlagerelemente vorübergehend entfernt. Anschließend können sie wieder montiert werden. Die Widerlagerelemente sind lediglich Beispiele. Sie werden in Abständen angeordnet, wobei naturgemäß nur ein Typ der gezeigten Elemente verwendet wird.

In den Figuren 11 und 12 ist ein Teil eines Klemmprofils 400 dargestellt mit einer oberen dachartigen schrägen Wand 402, einer Rückwand 404 sowie einer unteren Wand 406. Zwischen einer Abkantung 408 von der oberen Wand 402, die einen Vorsprung bildet und der unteren Wand 406 ist ein Eintrittsschlitz 410 gebildet. Das Einlageprofil ist hier nicht dargestellt.

Von unten ist an die untere Wand 406 ein Blechteil 412 angeschweißt. Das Blechteil fluchtet mit einer hinteren Kante 414 mit der Rückwand 404. Nach vom weist das Blechteil 412 eine bogenförmige Kante 416 auf. An der oberen Kante weist das Blechteil 412 eine Ausklinkung 418 auf bzw. eine Ausnehmung. Durch das Blechteil 412 erstreckt sich ein Stift 420, dessen Enden zu beiden Seiten über das Blechteil 412 überstehen. Der Stift erstreckt sich durch Klinken 422 auf jeder Seite des Blechteils 412, deren Kontur in Figur 11 zu erkennen ist. Die Klinken 422 haben ein Langloch 424, durch welches sich der Stift 420 jeweils hindurch erstreckt. Mit den Klinken 422 ist ein rechteckiger Widerlagerabschnitt 426 verschweißt, das in der in Figur 11 gezeigten Position in die Ausklinkung 418 hineinsteht. Wird der Widerlagerabschnitt 426 mit den Klinken angehoben, wird der Widerlagerabschnitt 426 aus der Klinkung herausgehoben und kann zusammen mit den Klinken 422 im Uhrzeigersinn verschwenkt werden, wie dies gestrichelt dargestellt ist. In dieser Position ist die dem Eintrittsschlitz 410 zugekehrte Fläche der unteren Wand 406 frei und ermöglicht das einfache Einsetzen des Einlegeprofils. Anschließend kann der gezeigte Klappmechanismus wieder in die in Figur 11 gezeigte Position zurückgeschwenkt werden, um ein Herausrutschen des Einlageprofils mit der Folie zu verhindern.

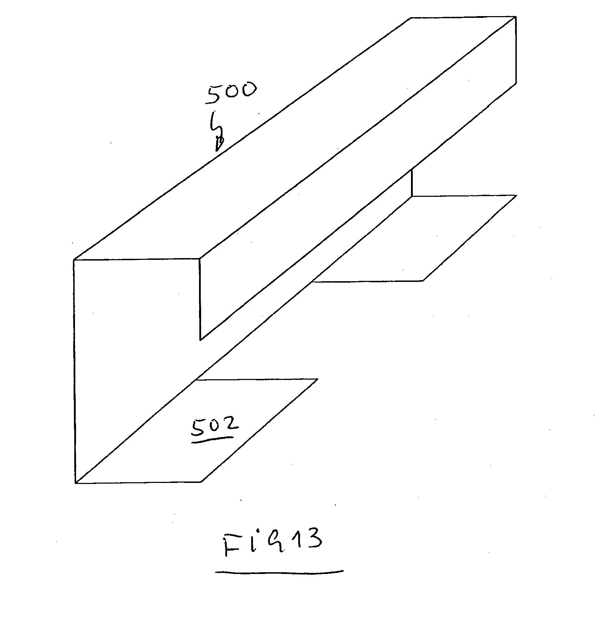

In Figur 13 ist ein Klemmprofil 500 dargestellt, das weitgehend dem nach Figur 10 gleicht. Allerdings ist die untere Wand in einzelne Wandabschnitt 402 unterteilt, die durch Abstände voneinander getrennt sind. Auf diese Weise lässt sich Material bei der Herstellung des Klemmprofils 500, z.B. beim Kanten vom Blechmaterial, einsparen.

## Patentansprüche

1. System aufweisend ein Einlegeprofil (126), mindestens eine Folie (130) und eine Behälterwand (14) eines Behälters, vorzugsweise aus Beton, insbesondere für Behälter zum Lagern und Gären von Gärstoffen, wobei die Behälterwand (14) nahe einem oberen Rand einen nach außen offenen Klemmkanal (112) mit Hinterschnitt für eine klemmende Aufnahme eines Randes der mindestens einen den Behälter an einer Oberseite abdeckenden Folie aus Kunststoff aufweist, wobei der Klemmkanal (112) einen verengten, nach außen offenen Eintrittsschlitz (124) aufweist, dessen Höhe kleiner ist als eine Höhe des in den Klemmkanal einsetzbaren, länglichen Einlageprofils (126), um welches die Folie (130) herumlegbar ist, wobei Querschnittsabmessungen von Klemmkanal (112), Eintrittsschlitz (124) und Einlageprofil (126) derart sind, dass das Einlageprofil (126) in einer ersten Stellung in den Klemmkanal (112) einführbar und in einer zweiten Stellung gegen ein Herausbewegen aus dem Klemmkanal gehalten ist, wenn die um das Einlageprofil herumgelegte Folie (130) unter Spannung steht, **dadurch gekennzeichnet, dass** der verengte Eintrittsschlitz (124) nur durch einen oberen Vorsprung begrenzt ist, während die annähernd ebene untere Wand des Klemmkanals (114) vorsprunglos in den Eintrittsschlitz (124) übergeht.

2. System nach Anspruch 1, **dadurch gekennzeichnet, dass** das Einlageprofil (126) eine Sollbruchstelle (132) aufweist, wobei das Einlageprofil (126) im Wesentlichen um eine horizontale Achse knickt, wenn an der Folie (130) eine vorgegebene Kraft angreift.

3. System nach Anspruch 1 oder 2, **dadurch gekennzeichnet, dass** das Einlageprofil aus einem Material hergestellt ist, das bei einer maximal zulässigen Kraft auf die Folie (130) durch Eigenverformung die kraftschlüssige Verbindung der Folie (130) gelockert oder gelöst wird.

4. System nach Anspruch 1, wobei in einem oberen Bereich der Behälterwand (14) ein ringförmiges Klemmprofil (300, 400, 500) angeordnet ist, das den inneren Klemmkanal mit einem nach außen offenen Eintrittsschlitz aufweist, wobei ferner der wandnahe Bereich der Folie (130) einen Winkel zur Horizontalen einschließt und das Klemmprofil (300, 400, 500) einen oberen und einen unteren Schenkel hat, der der Folie (130) zugewandte obere Schenkel des Klemmprofils (300, 400, 500) so geneigt ist, dass er sich bis zur maximalen zulässigen Zugbelastung der Folie (130) annähernd parallel oder in einem kleinen Winkel zur Folie (130) so erstreckt, dass eine von der Folie (130) auf den oberen Schenkel aufgebrachte Kraft zu ihrem größeren Teil als Druckkraft auf diesen Schenkel wirkt.

5. System nach einem der Ansprüche 1 bis 4, **dadurch gekennzeichnet, dass** der Klemmkanal (112) in das Material der Betonwand eingeformt ist.

6. System nach Anspruch 5, **dadurch gekennzeichnet, dass** eine Profilschiene (18a, 36, 200) zumindest teilweise in die Betonwand eingegossen ist.

7. System nach Anspruch 6, **dadurch gekennzeichnet, dass** die Profilschiene (18a, 36, 200) an der Behälterwand von außen befestigbar ist.

8. System nach Anspruch 6 oder 7, **dadurch gekennzeichnet, dass** eine obere Kante oder ein oberer Schenkel der Profilschiene (18a, 36, 200) mit der Oberseite der Behälterwand (14) bündig ist.

9. System nach Anspruch 6, **dadurch gekennzeichnet, dass** die Oberseite (44) der Behälterwand (14) zur Außenseite hin ansteigt.

10. System nach Anspruch 6, **dadurch gekennzeichnet, dass** die Profilschiene (36). mit einem Abstand unterhalb der Oberseite der Behälterwand (14) angeordnet und eine äußere Kante der Wandoberseite gerundet ist.

11. System nach Anspruch 4, **dadurch gekennzeichnet, dass** das Klemmprofil (300, 400, 500) aus zwei oder mehreren Schienenabschnitten zusammengesetzt ist.

12. System nach Anspruch 4, **dadurch gekennzeichnet, dass** das Klemmprofil (300, 400, 500) aus in der Längsebene geteilten Schienenabschnitten besteht, die im Stegbereich kraftschlüssig miteinander verbunden sind.

13. System nach einem der Ansprüche 1 bis 4, **dadurch gekennzeichnet, dass** der Klemmkanal (112) von einer verlorenen Schalung gebildet ist.

14. System nach Anspruch 13, **dadurch gekennzeichnet, dass** die verlorene Schalung schienenförmig ist bzw. aus Schienenabschnitten besteht.

15. System nach einem der Ansprüche 1 bis 14, **dadurch gekennzeichnet, dass** die untere Wand nahe dem Eintrittsschlitz (124) des Klemmkanals (112) in Längsabschnitten Erhebungen aufweist, die entfernbar oder beweglich so angeordnet sind, dass sie in einer ersten Position ein Widerlager für das im Klemmkanal (112) befmdliche Einlageprofil (126) bilden und in einer zweiten Position das Widerlager aus dem Eintrittsschlitz (124) entfernt ist.

16. System nach Anspruch 15, **dadurch gekennzeichnet, dass** in Öffnungen der unteren Wand (316) einsteckbare oder einschraubbare Widerlagerelemente (326, 328) vorgesehen sind.

17. System nach Anspruch 15, **dadurch gekennzeichnet, dass** an der unteren Wand (404) gelagerte Klappmechanismen (408) vorgesehen sind.

18. System nach Anspruch 15, **dadurch gekennzeichnet, dass** Klemmelemente (330) vorgesehen sind, die entfernbar von vom auf die untere Wand (316) aufklemmbar ausgebildet sind.

19. System nach einem der Ansprüche 4 bis 18, **dadurch gekennzeichnet, dass** die untere Wand aus in Längsrichtung des Klemmprofils (500) beabstandeten Wandabschnitten (502) besteht.

## Claims

1. A system having an insertion profile (126), at least one film (130) and a container wall (14) of a container, preferably made of concrete, in particular for containers for storing and fermenting fermented goods, wherein the container wall (14) has a clamping channel (112) that is open to the outside and adjacent to a top edge and has an undercut for a clamping seat of an edge of the at least one film made of plastic which covers the top side of the container, wherein the clamping channel (112) has a narrow entry slot (184) that is open to the outside and has a height that is less than a height of the elongated insertion profile (126) that can be inserted in the clamping channel and around which the film (130) can be placed, wherein the cross-sectional dimensions of the clamping channel (112), entry slot (124) and insertion profile (126) are such that the insertion profile (126) can be inserted into the clamping channel (112) in a first position, and can be held against moving out of the clamping channel in a second position when the film (130) placed around the insertion profile is under tension, **characterized in that** the narrow entry slot (124) is only bordered by a top projection, whereas the approximately flat bottom wall of the clamping channel (114) transitions without a projection into the entry slot (134).

2. The system according to claim 1, **characterized in that** the insertion profile (126) has a predetermined breaking point (132), wherein the insertion profile (126) kinks substantially about a horizontal axis when a predetermined force acts on the film (130).

3. The system according to claim 1 or 2, **characterized in that** the insertion profile is made of a material that, by means of intrinsic deformation, loosens or releases the friction-locked connection of the film (130) under a maximum permissible force upon the film (130).

4. The system according to claim 1, wherein an annular clamping profile (300, 400, 500) is arranged in a top region of the container wall (14) that has the inner clamping channel with an entry slot open to the outside, wherein furthermore, the region of the film (130) close to the wall encloses an angle to the horizontal, and the clamping profile (300, 400, 500) has an upper and a lower leg, and the upper leg of the clamping profile (300, 400, 500) facing the film (130) is angled such that it extends approximately parallel or at a slight angle to the film (130) up to the maximum permissible traction on the foil (130) such that the majority of the force exerted on the upper leg by the film (130) acts as compressive force on said leg.

5. The system according to one of claims 1 to 4, **characterized in that** the clamping channel (112) is formed in the material of the concrete wall.

6. The system according to claim 5, **characterized in that** a mounting rail (18a, 36, 200) is at least partially cast into the concrete wall.

7. The system according to claim 6, **characterized in that** the mounting rail (18a, 36, 200) can be fastened to the container wall from the outside.

8. The system according to claim 6 or 7, **characterized in that** a top edge or a top leg of the mounting rail (18a, 36, 200) is flush with the top side of the container wall (14).

9. The system according to claim 6, **characterized in that** the top side (44) of the container wall (14) rises toward the outside.

10. The system according to claim 6, **characterized in that** the mounting rail (36) is arranged at a distance below the top side of the container wall (14), and an outer edge of the wall top side is rounded.

11. The system according to claim 4, **characterized in that** the clamping profile (300, 400, 500) is composed of two or more rail sections.

12. The system according to claim 4, **characterized in that** the clamping profile (300, 400, 500) consists of rail sections divided in the longitudinal plane which are connected to each other by a force fit in the web region.

13. The system according to one of claims 1 to 4, **characterized in that** the clamping channel (112) is formed by a permanent formwork.

14. The system according to claim 13, **characterized in that** the permanent formwork is in the shape of a rail or consists of rail sections.

15. The system according to one of claims 1 to 14, **characterized in that** the bottom wall next to the entry slots (124) of the clamping channel (112) has elevations in longitudinal sections that are removable or arranged to be movable such that, in a first position, they form a stop for the insertion profile (126) located in the clamping channel (112) and, in a second position, the stop is removed from the entry slot (124).

16. The system according to claim 15, **characterized in that** stop elements (324, 328) are provided that can be inserted or screwed into openings in the bottom wall (316).

17. The system according to claim 15, **characterized in that** folding mechanisms (408) are provided which are mounted on the bottom wall (404).

18. The system according to claim 15, **characterized in that** clamping elements (330) are provided which are designed to be removable and clamp-fit onto the bottom wall (316) from the front.

19. The system according to one of claims 4 to 18, **characterized in that** the bottom wall consists of wall sections (502) which are spaced in the longitudinal direction of the clamping profile (500).

## Revendications

1. Système présentant un profilé d'insert (126), au moins un film (130) et une paroi de réservoir (14) d'un réservoir, de préférence en béton, en particulier pour des réservoirs destinés à l'entreposage et la fermentation de ferments, la paroi de réservoir (14) présentant, près d'un bord supérieur, un canal de serrage (112) ouvert vers l'extérieur, avec une contre-dépouille pour une réception serrante d'un bord du film en matière plastique au moins au nombre de un recouvrant le réservoir sur un côté supérieur, le canal de serrage (112) présentant une fente d'entrée (124) rétrécie ouverte vers l'extérieur dont la hauteur est plus petite qu'une hauteur du profilé d'insert (126) oblong pouvant être inséré dans le canal de serrage et autour duquel le film peut être placé, des dimensions de section transversale du canal de serrage (112), de la fente d'entrée (124) du profilé d'insert (126) étant telles que le profilé d'insert (126) peut, dans une première position, être introduit dans le canal de serrage (112) et, dans une deuxième position, être retenu contre une expulsion hors du canal de serrage quand le film (130) placé autour du profilé d'insert est sous tension, **caractérisé en ce que** la fente d'entrée (124) rétrécie n'est limitée que par une saillie supérieure tandis que la paroi inférieure approximativement plane du canal de serrage (114) se transforme sans saillie en fente d'entrée (124).

2. Système selon la revendication 1, **caractérisé en ce que** le profilé d'insert (126) présente un emplacement de rupture programmée (132), le profilé d'insert (126) se pliant essentiellement autour d'un axe horizontal quand une force prédéfinie s'exerce sur le film (130).

3. Système selon la revendication 1 ou 2, **caractérisé en ce que** le profilé d'insert est réalisé dans un matériau tel que, en présence d'une force maximale admissible sur le film (130), le raccordement par liaison de force du film (130) se relâche ou se desserre par déformation propre.

4. Système selon la revendication 1, un profilé de serrage (300, 400, 500) annulaire étant disposé dans une zone supérieure de la paroi de réservoir (14) présentant le canal de serrage intérieur avec une fente d'entrée ouverte vers l'extérieur, la zone du film (130) proche de la paroi faisant un angle par rapport à l'horizontale, et le profilé de serrage (300, 400, 500) ayant une branche supérieure et une branche inférieure, la branche supérieure du profilé de serrage (300, 400, 500) tournée vers le film (130) étant inclinée de telle sorte qu'elle s'étire jusqu'à la contrainte de traction admissible maximale du film (130) approximativement parallèlement au film (130) ou en faisant un petit angle avec celui-ci de telle sorte qu'une force appliquée par le film (130) à la branche supérieure agit pour sa plus grande part en tant que force de pression sur cette branche.

5. Système selon une des revendications 1 à 4, **caractérisé en ce que** le canal de serrage (112) est formé dans le matériau de la paroi en béton.

6. Système selon la revendication 5, **caractérisé en ce qu'**un rail profilé (18a, 36, 200) est coulé au moins partiellement dans la paroi en béton.

7. Système selon la revendication 6, **caractérisé en ce que** le rail profilé (18a, 36, 200) peut être fixé de l'extérieur sur la paroi en béton.

8. Système selon la revendication 6 ou 7, **caractérisé en ce qu'**une arête supérieure ou une branche supérieure du rail profilé (18a, 36, 200) affleure avec le côté supérieur de la paroi de réservoir (14).

9. Système selon la revendication 6, **caractérisé en ce que** la partie supérieure (44) de la paroi de réservoir (14) monte vers le côté extérieur.

10. Système selon la revendication 6, **caractérisé en ce que** le rail profilé (36) est disposé à une certaine distance au-dessous du côté supérieur de la paroi de réservoir (14) et **en ce qu'**une arête extérieure du côté supérieur de paroi est arrondie.

11. Système selon la revendication 4, **caractérisé en ce que** le profilé de serrage (300, 400, 500) est composé de deux ou de plusieurs tronçons de rail.

12. Système selon la revendication 4, **caractérisé en ce que** le profilé de serrage (300, 400, 500) est constitué de tronçons de rail divisés dans le plan longitudinal qui sont raccordés les uns aux autres par liaison de force dans la zone de leur âme.

13. Système selon une des revendications 1 à 4, **caractérisé en ce que** le canal de serrage (112) est formé d'un coffrage perdu.

14. Système selon la revendication 13, **caractérisé en ce que** le coffrage perdu est en forme de rail ou respectivement est constitué de tronçons de rail.

15. Système selon une des revendications 1 à 14, **caractérisé en ce que** la paroi inférieure présente, près de la fente d'entrée (124) du canal de serrage (112), des surélévations dans des tronçons longitudinaux qui sont disposées de façon amovible ou mobile de telle façon que, dans une première position, elles forment une butée pour le profilé d'insert (126) situé dans le canal de serrage (112) et de telle façon que, dans une deuxième position, la butée est enlevée de la fente d'entrée (124).

16. Système selon la revendication 15, **caractérisé en ce qu'**il est prévu des éléments de butée (326, 328) pouvant être enfichés ou vissés dans des ouvertures de la paroi inférieure (316).

17. Système selon la revendication 15, **caractérisé en ce qu'**il est prévu des mécanismes de rabat (408) supportés sur la paroi inférieure (404).

18. Système selon la revendication 15, **caractérisé en ce qu'**il est prévu des éléments de serrage (330) qui sont constitués de façon à pouvoir être serrés sur la paroi inférieure (316) de façon à pouvoir être enlevés par l'avant.

19. Système selon une des revendications 4 à 18, **caractérisé en ce que** la paroi inférieure est constituée de tronçons de paroi (502) espacés dans la direction longitudinale du profilé de serrage (500).
